# EUROPÄISCHE PATENTSCHRIFT

(11) **EP 3 531 964 B1**
(45) Veröffentlichungstag und Bekanntmachung des Hinweises auf die Patenterteilung: **30.06.2021**
(21) Anmeldenummer: 17791998.2
(22) Anmeldetag: 16.10.2017
(51) Int. Cl.: A61C 19/045, A61C 7/12, A61B 5/11, A61B 5/00

(54) **VERFAHREN UND VORRICHTUNG ZUR AUFNAHME VON KIEFERBEWEGUNGEN**
METHOD AND DEVICE FOR RECORDING JAW MOVEMENTS
PROCÉDÉ ET DISPOSITIF POUR ENREGISTRER LES MOUVEMENTS MANDIBULAIRES

(30) Priorität: 27.10.2016 DE 102016120583
(43) Veröffentlichungstag der Anmeldung: 04.09.2019
(73) Patentinhaber: SICAT GmbH & Co. KG, 53175 Bonn (DE)
(72) Erfinder: HANSSEN, Nils, 53125 Bonn (DE); KUSCH, Jochen, 53343 Wachtberg (DE); LEHNER, Tobias, 53229 Bonn (DE)
(74) Vertreter: Braun-Dullaeus Pannen Emmerling Patent- & Rechtsanwaltspartnerschaft mbB
(86) Internationale Anmeldenummer: PCT/EP2017/076303
(87) Internationale Veröffentlichungsnummer: WO 2018/077643

(56) Entgegenhaltungen:
- DE-A1- 3 500 605
- DE-A1- 10 236 333
- US-A- 4 842 519
- US-A- 5 143 086
- YOON H-J ET AL: "Kinematic study of the mandible using an electromagnetic tracking device and custom dental appliance: Introducing a new technique", JOURNAL OF BIOMECHANICS, PERGAMON PRESS, NEW YORK, NY, US, Bd. 39, Nr. 12, 31. Januar 2006 (2006-01-31), Seiten 2325-2330, XP024980434, ISSN: 0021-9290, DOI: 10.1016/J.JBIOMECH.2005.07.001 [gefunden am 2006-01-01]
- ARNE WAGNER ET AL: "A comparative analysis of optical and conventional axiography for the analysis of temporomandibular joint movements", JOURNAL OF PROSTHETIC DENTISTRY., Bd. 90, Nr. 5, 30. November 2003 (2003-11-30), Seiten 503-509, XP055441700, AMSTERDAM, NL ISSN: 0022-3913, DOI: 10.1016/S0022-3913(03)00482-7

## Beschreibung

Die Erfindung betrifft ein Verfahren sowie eine Vorrichtung zur Aufnahme von Kieferbewegungen eines Patienten, wobei ein Sensorelement und ein für das Sensorelement erkennbarer Positionsmarker am Kopf des Patienten angebracht werden. Hierbei kann im Prinzip frei gewählt werden, ob das Sensorelement an den Kopf und der Positionsmarker an den Unterkiefer angebracht wird oder umgekehrt. Die Kieferbewegungen des Patienten werden durch die Relativbewegung des Sensorelementes zum Positionsmarker erfasst und aufgenommen.

Aus den DE 102 36 333 A1, DE 10 2014 111 643 A1 und DE 20 2013 001 910 U1 sind derartige Verfahren respektive Vorrichtungen bekannt. Dabei werden Bissgabeln adhäsiv oder mittels eines Registriermaterials an den Zähnen befestigt.

Die US 5,143,086 beschreibt eine Vorrichtung zum Bestimmen der Unterkieferbewegung, wobei drei Leuchtdioden an den Ecken eines Dreiecks vorübergehend an den Zähnen des Gegenstands befestigt sind, die Leuchtdioden sequentiell erregt werden und zwei Sensoren, die auf die Leuchtdioden ansprechen, an einem Kopfstück angebracht sind und die Bewegung detektieren

Aus dem DE 11 2005 000 700 T5 ist eine Vorrichtung und ein Verfahren zum Messen einer dreidimensionalen Kieferbewegung bekannt, bei dem Sensoren adhäsiv an den Zähnen befestigt werden.

Aus den Dokumenten DE 10 2014 225 457 A1, WO 2014/186 278 A1, WO 2007/133 422 A2 und WO 2003/096 922 A1 sind kieferorthopädische Vorrichtungen bekannt, bei denen Sensoren in oder an den Zahnklammern befestigt sind

Bei den in der Praxis bekannten Verfahren und Vorrichtungen werden zur Messung von Kaubewegungen eines Patienten mechanische, temporär rigide Verbindungen zwischen dem Mess-System und festen Strukturen des Unterkiefers, beispielsweise den Knochen oder den Zähnen, hergestellt. Bei den bekannten Mess-Systemen ist typischerweise das Sensorelement fest mit den Strukturen des oberen Kopfbereiches des Patienten verbunden, indem dem Patienten beispielsweise eine Art Bügelhalterung aufgesetzt wird, wobei der Positionsmarker fest mit den Zähen des Unterkiefers verbunden ist. Um den Positionsmarker fest mit dem Unterkiefer zu verbinden, wird ein sogenanntes paraokklusales Attachement verwendet. Dies ist im Prinzip eine an die Biegung des Zahnbogens des Patienten angepasste Halterung, die mittels einer ausgehärtet Masse bzw. Klebers an den Vestibulärflächen der Zähne befestigt wird.

Nachteilig bereitet häufig bereits die Befestigung des paraokklusales Attachements mittels der ausgehärteten Masse bzw. des Klebers dem Zahnarzt Probleme, da ein Kompromiss zwischen genügend Halt des Positionsmarkers an den Zähnen während der Aufnahme der Kieferbewegung durch das Mess-System und einer einfachen Ablösung des paraokklusales Attachement nach erfolgter Messung gefunden werden muss. Dies erfordert Erfahrung und die Verwendung geeigneter Werkstoffe. Durch die Glattheit der Zähne und Reste von Speichel gelingt die Befestigung des paraokklusalen Attachements auch erfahrenen Zahnärzten oft nur unzureichend. Bei Patienten mit einem Überbiss werden die Vestibulärflächen der Unterkieferzähne in einem Schlussbiss häufig so stark verdeckt, dass eine Befestigung des paraokklusalen Attachements schwierig ist. Durch das Gewicht des Mess-Systems, dass an den Teller des paraokklusales Attachement befestigt wirkt, wirken hohe Drehmomente an den Vestibulärflächen der Zähne, weshalb eine starke Haftung benötigt wird, insbesondere wenn der Unterkiefer während der Messung schnelle Bewegungen ausführt. In der bisherigen Praxis werden die geklebten Attachments nachteilig als eine große Klebefläche auf einmal von den Zähnen abgelöst, was einen hohen Kraftaufwand erfordert und dadurch auch ein potentielles Verletzungsrisiko für den Patienten darstellt.

Es ist die Aufgabe der Erfindung ein Verfahren zur Messung von Kieferbewegungen anzugeben bei dem ein Sensorelement oder ein Positionsmarker eines Mess-Systems einfach und zuverlässig an den Zähnen des Unterkiefers des Patienten lösbar befestigt wird. Ferner ist die Aufgabe der Erfindung eine Vorrichtung für das Verfahren bereitzustellen. Die Aufgabe wird erfindungsgemäß durch ein Verfahren mit den Merkmalen des unabhängigen Anspruchs eins und eine Vorrichtung mit den Merkmalen des unabhängigen Anspruchs fünf gelöst. Vorteilhafte Ausführungsformen der Erfindung sind in den Unteransprüchen genannt.

Der bei dem erfindungsgemäßen Verfahren realisierte Kerngedanke liegt darin, dass auf Oberflächen der Zähne des Unterkiefers Zahnklammern lösbar aufgeklebt werden, wobei das Sensorelement oder der Positionsmarker vermittels der Zahnklammern befestigt wird.

Hierbei werden die Zahnklammern auf Oberflächen der Zähne lösbar an Befestigungspunkten aufgeklebt werden, wobei das Sensorelement oder der Positionsmarker zumindest abschnittsweise aus dem Mundraum des Patienten herausragt. Die Zahnklammern stellen eine feste Verbindung zwischen dem Sensorelement oder dem Positionsmarker des Mess-Systems und den Zähnen her, die auch großen Drehmomenten standhält. Zusätzlich weisen Zahnarztpraxen langjährige Erfahrungen bei der Verwendung von Zahnklammern auf, wie sie typischerweise bei Zahnspangen verwendet werden. Die Zahnklammern gewähren nach erfolgter Messung mit einem speziellen Werkzeug eine gute Lösbarkeit von den Zähnen des Patienten. Vorteilhaft wird bei dem erfindungsgemäßen Verfahren jede Zahnklammer schonend einzelnen von den Zähnen des Patienten abgelöst. Es existieren gebrauchsfertige Kits, um die Zahnklammern sicher auf die Zähne zu kleben und zu entfernen.

Bevorzugt werden die Befestigungspunkte spiegelsymmetrisch zur spiegelsymmetrischen Achse des Unterkiefers angebracht. Dies sorgt für eine gleichmäßige Belastung des Unterkiefers durch die wirkenden Drehmomente, sodass sowohl die Zahnklammern möglichst gleichmäßig belastet werden als auch das subjektive Wohlbefinden des Patienten hierdurch gesteigert wird. Zweckmäßig werden die Zähne für die Befestigungspunkte frei gewählt. Unter zahntechnischen Aspekten ist unter Umständen eine Abweichung von der spiegelsymmetrischen Anordnung der Befestigungspunkte sinnvoll bzw. notwendig. Fehlen dem Patienten beispielsweise auf einer Seite des Unterkiefers Zähne, muss von der spiegelsymmetrischen Anordnung abgewichen werden. Auch bei Erkrankungen eines spiegelsymmetrisch vorhandenen Zahns kann der Zahnarzt einen anderen Befestigungspunkt an einem anderen Zahn seiner Wahl frei festlegen, um den erkrankten Zahn zu schonen.

In einer Vorgehensweise, die nicht zur beanspruchten Erfindung gehört, wird ein Behandlungsdraht lösbar an den Zahnklammern befestigt, wobei jeweils zwei Zahnklammern horizontal durch den Behandlungsdraht verbunden werden, wobei das Sensorelement oder der Positionsmarker vermittels des Behandlungsdrahtes lösbar zwischen zwei Zahnklammern befestigt wird. Auf diese Weise kann das Sensorelement oder der Positionsmarker vorteilhaft auch während einer laufenden kieferorthopädische Behandlung, wie beispielsweise bei einer Zahnspangenbehandlung, sicher am Unterkiefer befestigt werden, indem die Zahnklammern verwendet werden, die bereits zum Zwecke der kieferorthopädische Behandlung durch die Zahnspange an die Zähne des Patienten angebracht sind.

Zweckmäßig verlaufen mehrere Behandlungsdrähte durch die Zahnklammern, wobei das Sensorelement oder der Positionsmarker an der Mehrzahl von Behandlungsdrähten befestigt wird. Durch die Verwendung von mehreren Behandlungsdrähten in verschiedenen Höhen an denen das Sensorelement oder der Positionsmarker befestigt wird, ist eine zusätzliche Stabilität gegenüber einer Rotationsbewegung des Sensorelementes oder des Positionsmarkers um den Behandlungsdraht gewährt.

Bevorzugt wird ein erstes Ende eines Trageelements lösbar an den Zahnklammern befestigt, wobei ein zweites Ende des Tragelements lösbar an das Sensorelement oder den Positionsmarker befestigt wird. Diese zweiteilige Ausgestaltung des am Unterkiefer des Patienten befestigten Teils des Mess-Systems bietet vorteilhaft die Möglichkeit, dass das Sensorelement oder der Positionsmarker erst in einem letzten Schritt, nachdem die Fixierung des Trageelementes am Unterkiefer des Patienten vollzogen ist, angebracht wird. Dies erleichtert die Befestigung bzw. die Justage und verhindert beispielsweise auch, dass das Sensorelement oder der Positionsmarker beim Befestigungsvorgang beschädigt wird.

Gemäß einem weiteren Aspekt der Erfindung wird eine Messvorrichtung zur Aufnahme von Kieferbewegungen des Patienten nach einem der vorhergehend beschriebenen Verfahren bereitgestellt, umfassend ein Mess-System, das ein Sensorelement und einen Positionsmarker aufweist, Zahnklammern, die auf einer Seite mit einem lösbaren Klebstoff an die Zähnen des Unterkiefers angebracht sind und auf der anderen Seite eine Aufnehmung aufweisen, wobei das Sensorelement oder der Positionsmarker lösbar befestigbar ist.

Bevorzugt wird das Sensorelement oder der Positionsmarker durch ein passendes Aufnehmungsgegenstück lösbar an den Aufnehmungen der Zahnklammern befestigt. Vorteilhaft ist es hierdurch möglich, das Sensorelement oder den Positionsmarker ohne zusätzliche Klebemittel mechanisch fest in die Aufnehmung einzubringen, und dass das Sensorelement oder der Positionsmarker durch das Ausführen bestimmter Arbeitsschritte mechanisch wieder aus der Aufnehmung gelöst werden kann. Die entsprechenden Bauteile werden hierdurch zerstörungsfrei voneinander getrennt, sodass diese nach einer Desinfektion wiederverwendbar sind.

Vorzugsweise ist der Behandlungsdraht lösbar in den Aufnehmungen fixiert und verläuft horizontal zwischen jeweils zwei Zahnklammern, wobei das Sensorelement oder der Positionsmarker lösbar mittels Drahtklammern an dem Behandlungsdraht befestigt ist. Die Drahtklammern ermöglichen es hierbei vorteilhaft das Sensorelement oder den Positionsmarker an die Behandlungsdrähte einer schon vorhandenen Zahnspange zu fixieren. Die Drahtklammern können beispielsweise an den Behandlungsdraht geclipst, verrastet, verschraubt oder auf andere Art und Weise angebracht werden.

Gemäß einer Vorgehensweise, die nicht zur beanspruchten Erfindung gehört, wird eine Verwendung der offenbarten Vorrichtung für eine kieferorthopädische Behandlung an den Zähnen des Unterkiefers eines Patienten angegeben, wobei eine lösbar gehaltenen Zahnklammer ("Bracket") zur Befestigung einer Messvorrichtung genutzt wird. Vorteilhaft kann also eine herkömmliche Zahnspannung zur Befestigung des Mess-Systems verwendet werden. Auf diese Weise kann je nach Umständen eine schon am Patienten angebracht Zahnspange verwendet werden oder die Zahnspange eigens für die Messvorrichtung angebracht werden.

Gemäß einer bevorzugten Ausgestaltung ist das Sensorelement oder der Positionsmarker mittels eines Trageelements lösbar mit den Zahnklammern oder den Drahtklammern verbunden.

Weitere Vorteile, Eigenschaften und Weiterbildungen der Erfindung ergeben sich aus den abhängigen Ansprüchen sowie aus der nachstehenden Beschreibung von bevorzugten Ausführungsbeispielen.

Die Erfindung wird im Folgenden unter Bezugnahme auf die Zeichnungen anhand der bevorzugten Ausführungsbeispiele näher erläutert.
- Fig. 1:: zeigt einen Patienten, der eine erfindungsgemäße Messvorrichtung zur Erfassung von Kieferbewegungen an seinem Kopf trägt.
- Fig. 2:: zeigt ein erstes erfindungsgemäßes Verfahren zur Befestigung eines Positionsmarkers der Messvorrichtung an Zähnen des Patienten.
- Fig. 3:: zeigt eine erste schematische Ausgestaltung der Befestigung des Positionsmarkers an Behandlungsdrähten einer Zahnspange zu Illustrationszwecken.
- Fig. 4:: zeigt eine zweite schematische Ausgestaltung der Befestigung des Positionsmarkers an Behandlungsdrähten der Zahnspange zu Illustrationszwecken.
- Fig. 5:: zeigt ein zweites erfindungsgemäßes Verfahren zur Befestigung des Positionsmarkers an Behandlungsdrähten der Zahnspange des Patienten.
- Fig. 6:: zeigt mehrere Behandlungsdrähte an einem Kopplungselement.
- Fig. 7:: zeigt mehrere frei wählbare Befestigungspunkte an dem Kopplungselement.

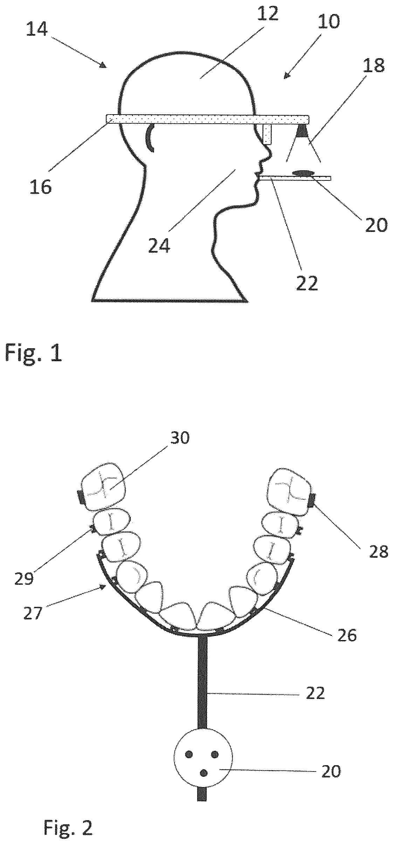

Fig. 1 zeigt wie das Mess-System 10 der Messvorrichtung am Kopf 12 eines Patienten 14 angebracht ist. Vorliegend ist das Mess-System 10 zweistückig ausgebildet und weist ein Sensorelement 18 und einen Positionsmarker 20 auf, wobei der Positionsmarker 20 auf ein Trageelement 22 aufgebracht ist, welches zumindest teilweise in den Mundraum 24 des Patienten eingebracht ist und dort an dessen Zähnen 30 des Unterkiefers befestigt wird. Das Sensorelement 18 des Mess-Systems 10 ist mittels einer Bügelhalterung 16 an den Kopf 12 des Patienten 14 angebracht und weist einen möglichst kleinen Bewegungsspielraum auf, sodass aus der Relativbewegung zwischen dem Positionsmarker 20 und dem Sensorelement 18 auf die Kieferbewegung des Patienten 14 geschlossen werden kann und fehlerhafte Einflüsse minimiert werden.

Das Mess-System 10 kann zweckmäßigerweise auch einstückig ausgebildet sein, wobei hierbei bevorzugt das Trageelement 22 und die Bügelhalterung 16 durch ein Gelenk verbunden sind. Ebenso können der Positionsmarker 20 und das Trageelement 22 bevorzugt als ein einstückig ausgebildeter Positionsmarker 20 vorliegen. Die zweistückige Ausbildung bietet den Vorteil, dass der Positionsmarker 20 nach dem Anbringen des Trageelements 22 an den Zähne 30 des Patienten 14 noch verschoben und dadurch feinjustiert werden kann. Bewegt der Patient 14 nun seinen Unterkiefer, folgt der Positionsmarker 20 dieser Bewegung, wobei die entstehende Bewegungskurve durch das Sensorelement 18 aufgezeichnet und in einem Computer zur späteren Auswertung abgespeichert wird.

Fig. 2 zeigt ein erstes erfindungsgemäßes Verfahren zur Befestigung des Positionsmarkers 20 der Messvorrichtung an den Zähnen 30 des Unterkiefers des Patienten 14. Hierbei werden Zahnklammern 28 an einer ersten Seite durch den Zahnarzt an ausgewählten Befestigungspunkten 27 an den Vestibulärflächen der Zähne 30 angeklebt. Als Vestibulärflächen werden die der Mundöffnung des Patienten 14 zugewandten Oberflächenseiten der Zähne 30 bezeichnet. An einer zweiten, den Zähnen 30 abgewandten Seite der Zahnklammern 28 sind Aufnehmungen 29 vorgesehen, um andere Bauteile an den Zahnklammer 28 befestigen zu können. In Fig. 2 ist ein Kopplungselement 26 gezeigt, welches passende Aufnehmungsgegenstücke aufweist und mechanisch mittels der Aufnehmungsgegenstücke an den Aufnehmungen 29 durch den Zahnarzt befestigt wird. Zweckmäßig wird das Kopplungselement 26 hierbei mittels vorher definierter Arbeitsschritte mechanisch und ohne Klebstoff in den Aufnehmungen 29 fixiert und kann durch nachfolgende Arbeitsschritte, nach der Aufzeichnung der Kaubewegung, wieder zerstörungsfrei entfernt werden.

Das Kopplungselement 26 kann durch den Zahnarzt leicht verbogen werden, sodass die äußere Formgebung der Kopplungseinheit 26 der des Zahnbogens des Patienten 14 entspricht. In Fig. 2 sind die Befestigungspunkte 27 spiegelsymmetrisch zur spiegelsymmetrischen Achse durch den Unterkiefer angeordnet. Das Trageelement 22 ist mit seinem einen Ende fest an das Kopplungselement 26 angebracht, wobei an dem den Zähnen 30 abgewandten Ende des Trageelements 22 der Positionsmarker 20 aufgebracht ist, sodass die Bewegung des Positionsmarkers 20 an die Bewegung des Unterkiefers gekoppelt ist. Die Kopplungseinheit 26 ist in Fig. 2 dadurch fest an den Zähnen 30 des Patienten 14 fixiert, sodass auch größere Drehmomente, wie sie beispielsweise durch die Kieferbewegung des Patienten 14 entstehen, kein Wackeln oder Verrutschen des Positionsmarkers 20 bedingen und die Kaubewegungen präzise aufgezeichnet werden.

Die Befestigungspunkte 27, die Form der Zahnklammern 28 und die Aufnehmungen 29 können mittels bildgebender Verfahren bestimmt oder vorgegeben werden, sodass die Aufnehmungsgegenstücke und das Kopplungselement 26 im Vorfeld entsprechend passend angefertigt werden können. Das Kopplungselement 26 wird beispielsweise durch ein Rapid-Prototyping Verfahren gefertigt, gefräst oder gedruckt, sodass es form- und/oder kraftschlüssig anliegt.

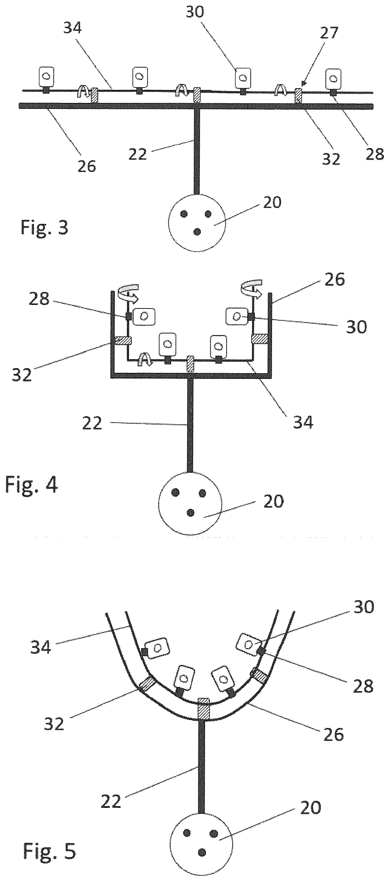

Fig. 3 zeigt eine erste schematische Ausgestaltung zur Befestigung des Positionsmarkers 20 an Behandlungsdrähten 26 einer Zahnspange zu Illustrationszwecken. Das Kopplungselement 26 ist in dieser Ausgestaltung nicht direkt mit den Zahnklammern 28 der Zähne 30 verbunden, sondern mittels Drahtklammern 32, welche an einen Behandlungsdraht 34 angebracht werden, der durch die Zahnklammern 28 verläuft und diese hierdurch verbindet. Ein bekanntes Beispiel einer solchen Anordnung ist in der zahntechnischen Praxis durch eine herkömmliche Zahnspange gegeben. Der Behandlungsdraht 34 wird mechanisch an den Zahnklammern 28 fixiert, wobei die Drahtklammern 32, welche an ihrem einen Ende fest mit dem Kopplungselement 26 verbunden sind an ihrem anderen Ende mechanisch mit dem Behandlungsdraht 34 fixiert werden. Zweckmäßigerweise können die Drahtklammern 32 hierbei an den Behandlungsdraht 34 geclipst, verrastet oder verschraubt und zu einem späteren Zeitpunkt wieder einfach entfernt werden. In dieser ersten Ausgestaltung definiert der Behandlungsdraht 34 durch seine gerade Erstreckung eine einzige Rotationsachse. Dies hat zur Folge, dass ein Drehmoment, welches auf das Trageelement 22 bzw. den Positionsmarker 20 wirkt, leicht zu einer Verdrehung des Positionsmarkers 22 in Bezug auf diese Rotationsachse führt.

In Fig. 4 wird gezeigt, wie durch einen rechteckigen Verlauf des einstückig ausgebildeten Behandlungsdrahts 34 insgesamt drei Rotationsachsen ausgebildet werden, die sich durch die einstückige Ausgestaltung des Behandlungsdrahts 34 bezüglich ihrer jeweiligen Rotation gegenseitig in einer Art hemmen, sodass ein Drehmoment, das auf das Trageelement 22 wirkt, keine Rotation des Trageelements 22 zur Folge hat. Der Positionsmarker 20 ist also auch bei einer mittelbaren Anbringung an die Zahnklammern 28 mittels des Behandlungsdrahts 34 und der Drahtklammern 32 fixiert, sodass die Kieferbewegungen präzise aufgezeichnet werden können.

Fig. 5 zeigt ein zweites erfindungsgemäßes Verfahren zur Befestigung des Positionsmarkers 20 mittels des Behandlungsdrahts 34 an einer Zahnspange des Patienten 14. In geometrischer Hinsicht ist Fig. 5 zwischen den zur Illustration verwendeten Extremfällen von Fig. 3 und Fig. 4 angeordnet. Die an den Zahnbogen angepasste Biegung des Kopplungselements 26 und des Behandlungsdrahts 34 führt in der Art von Fig. 4 dafür dazu, dass der Positionsmarker 20 trotz einwirkende Drehmomente an den Unterkiefer des Patienten 14 fixiert ist und nicht rotiert.

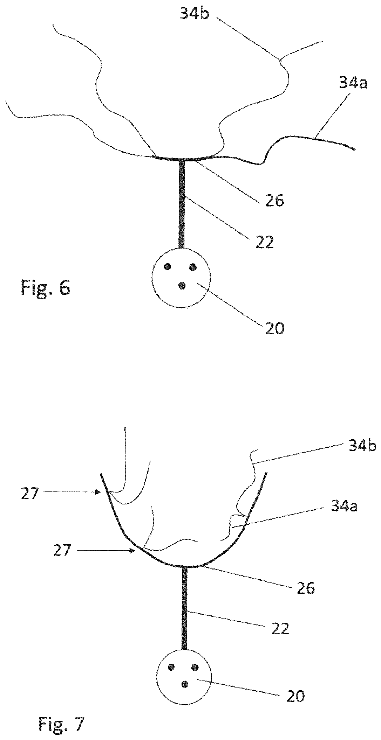

Fig. 6 zeigt mehrere Behandlungsdrähte 34a, b, die an einem Kopplungselement 26 angebracht sind und von diesem abgehen. Die Verwendung von mehreren Behandlungsdrähten 34a, b anstatt eines einzigen Behandlungsdrahtes 34, ermöglicht eine noch stärkere Befestigung des Positionsmarkers 20 am Unterkiefer des Patienten 14. Der behandelnde Arzt entscheidet zweckmäßigerweise im Einzelfall, ob die Verwendung von mehreren Behandlungsdrähten 34a, b bei einem bestimmten Patienten 14 notwendig ist.

Zusätzlich zu den mehreren Behandlungsdrähten 34a, b zeigt Fig. 7 eine frei wählbare Ausgestaltung der Befestigungspunkte 27 an dem Kopplungselement 26. Dies ist unter Umständen notwendig, falls der Patient 14 kranke Zähne 30 hat an denen zur Schonung kein Drehmoment angreifen soll oder falls dem Patienten 14 Zähne fehlen, sodass an diesen Stellen keine Zahnklammern 28 anbringbar sind. Der Zahnarzt entscheidet patientenindividuell wie er die Befestigungspunkte 27 auswählt, um eine ausreichende Befestigung des Positionsmarkers 20 am Unterkiefer des Patienten 14 sicherzustellen.

## Patentansprüche

1. Verfahren zur Aufnahme von Kieferbewegungen eines Patienten (14), wobei ein Sensorelement (18) und ein für das Sensorelement erkennbarer Positionsmarker (20) am Kopf (12) des Patienten (14) angebracht werden, wobei das Sensorelement (18) am Schädel und der Positionsmarker (20) am Unterkiefer, oder umgekehrt, angebracht werden, wobei bei einer Kieferbewegung die Relativbewegung des Sensorelements (18) zum Positionsmarker (20) aufgenommen wird,
**dadurch gekennzeichnet,**
**dass** auf Oberflächen der Zähne (30) des Unterkiefers Zahnklammern (28) lösbar aufgeklebt werden, wobei das Sensorelement (18) oder der Positionsmarker (20) vermittels der Zahnklammern (28) befestigt wird.

2. Verfahren nach Anspruch 1, **dadurch gekennzeichnet,**
**dass** das Sensorelement (18) oder der Positionsmarker (20) lösbar an Befestigungspunkten (27) der Zahnklammern (28) befestigt wird.

3. Verfahren nach Anspruch 1 oder 2, **dadurch gekennzeichnet,**
**dass** die Befestigungspunkte (27) spiegelsymmetrisch zur Achse des Unterkiefers angebracht werden.

4. Verfahren nach einem der vorhergehenden Ansprüche, **dadurch gekennzeichnet,**
**dass** ein erstes Ende eines Trageelements (22) lösbar an den Zahnklammern (28) befestigt wird und dass ein zweites Ende des Trageelements (22) lösbar an das Sensorelement (18) oder den Positionsmarker (20) befestigt wird.

5. Messvorrichtung zur Aufnahme von Kieferbewegungen eines Patienten (14) nach einem Verfahren der vorhergehenden Ansprüche, umfassend
ein Mess-System (10), das ein Sensorelement (18) und einen Positionsmarker (20) aufweist,
**gekennzeichnet durch** Zahnklammern (28), die mit einem Klebstoff lösbar an die Zähne (30) des Unterkiefers anbringbar sind und die jeweils eine Aufnehmung (29) aufweisen, an der das Sensorelement (18) oder der Positionsmarker (20) lösbar befestigbar ist.

6. Messvorrichtung nach Anspruch 5, **dadurch gekennzeichnet, dass** das Sensorelement (18) oder der Positionsmarker (20) durch ein passendes Aufnehmungsgegenstück lösbar an den Aufnehmungen (29) der Zahnklammern (28) befestigt ist.

7. Messvorrichtung nach Anspruch 5, **dadurch gekennzeichnet, dass** ein Behandlungsdraht (34) lösbar in den Aufnehmungen (29) fixiert ist und horizontal zwischen den Zahnklammern (28) verläuft, und dass das Sensorelement (18) oder der Positionsmarker (20) lösbar mittels Drahtklammern (32) an dem Behandlungsdraht (34) befestigt ist.

8. Messvorrichtung nach einem der Ansprüche 6 bis 7, **dadurch gekennzeichnet, dass** das Sensorelement (18) oder der Positionsmarker (20) mittels eines Trageelements (22) lösbar mit den Zahnklammern (28) oder dem Behandlungsdraht (34) verbunden ist.

## Claims

1. Method of recording jaw movements of a patient (14), wherein a sensor element (18) and a position marker (20) detectable for the sensor element are applied on the head (12) of the patient (14), wherein the sensor element (18) is applied on the skull and the position marker (20) on the lower jaw, or vice versa, wherein during a jaw movement the relative movement of the sensor element (18) in relation to the position marker (20) is recorded,
**characterised in**
**that** dental braces (28) are detachably adhered to surfaces of the teeth (30) of the lower jaw, wherein the sensor element (18) or the position marker (20) is attached by means of the dental braces (28).

2. Method according to claim 1, **characterised in that**
the sensor element (18) or the position marker (20) are detachably fastened to fastening points (27) of the dental braces (28).

3. Method according to claim 1 or 2 **characterised in that**
the fastening points (27) are applied mirror-symmetrically to the axis of the lower jaw.

4. Method according to any one of the preceding claims **characterised in that**
a first end of a support element (22) is detachably fastened to the dental braces (28) and **in that** a second end of the support element (22) is detachably fastened to the sensor element (18) or the position marker (20).

5. Measuring device for recording jaw movements of a patient (14) in accordance with a method of the preceding claims, having
a measuring system (10), that comprises a sensor element (18) and a position marker (20),
**characterised by** dental braces (28) which can be detachably applied with an adhesive to the teeth (30) of the lower jaw and which each have a receiving means (29) on which the sensor element (18) or position marker (20) can be detachably fastened.

6. Measuring device according to claim 5, **characterised in**
**that** the sensor element (18) or the position marker (20) is detachably fastened to the receiving means (29) of the dental braces (28) by way of a matching receiving means counter piece.

7. Measuring device according to claim 5 **characterised in**
**that** a treatment wire (34) is detachably affixed in the receiving means (29) and extends horizontally between the dental braces (28) and in that the sensor element (18) or the position marker (20) is detachably fastened to the treatment wire (34) by means of wire clips (32).

8. Measuring device according to any one of claims 6 to 7 **characterised in**
**that** the sensor element (18) or the position marker (20) is detachably connected by means of a support element (22) to the dental braces (28) or to the treatment wire (34).

## Revendications

1. Procédé d'enregistrement de mouvements de la mâchoire d'un patient (14), dans lequel un élément détecteur (18) et un marqueur de position (20) détectable par l'élément détecteur sont installés au niveau de de la tête (12) du patient (14), l'élément détecteur (18) étant installé au niveau du crâne et le marqueur de position (20) au niveau de la mâchoire inférieure ou inversement, le mouvement relatif de l'élément détecteur (18) par rapport au marqueur de position (20) lors d'un mouvement de la mâchoire étant enregistré,
**caractérisé en ce que**,
sur des surfaces des dents (30) de la mâchoire inférieure, des appareils dentaires (28) sont collés de manière dissociable, l'élément détecteur (18) ou le marqueur de position (20) étant fixé au moyen des appareils dentaires (28).

2. Procédé selon la revendication 1, **caractérisé en ce**
**que** l'élément détecteur (18) ou le marqueur de position (20) est fixé de manière dissociable à des points de fixation (27) des appareils dentaires (28).

3. Procédé selon la revendication 1 ou 2, **caractérisé en ce**
**que** les points de fixation (27) sont installés en symétrie miroir par rapport à l'axe de la mâchoire inférieure.

4. Procédé selon une des revendications précédentes, **caractérisé en ce**
**qu'**une première extrémité d'un élément porteur (22) est fixée de manière dissociable aux appareils dentaires (28) et qu'une seconde extrémité de l'élément porteur (22) est fixée de manière dissociable à l'élément détecteur (18) ou au marqueur de position (20).

5. Dispositif de mesure pour l'enregistrement de mouvements de la mâchoire d'un patient (14) suivant un procédé selon les revendications précédentes, comprenant
un système de mesure (10) qui présente un élément détecteur (18) et un marqueur de position (20),
**caractérisé par** des appareils dentaires (28) qui peuvent être installés de manière dissociable avec une colle au niveau des dents (30) de la mâchoire inférieure et qui présentent respectivement un support (29) auquel l'élément détecteur (18) ou le marqueur de position (20) peut être fixé de manière dissociable.

6. Dispositif de mesure selon la revendication 5, **caractérisé en ce**
**que** l'élément détecteur (18) ou le marqueur de position (20) est fixée par une contre-pièce de réception appropriée de manière dissociable aux supports (20) des appareils dentaires (28).

7. Dispositif de mesure selon la revendication 5, **caractérisé en ce**
**qu'**un fil de traitement (34) est fixé de manière dissociable dans les supports (29) et s'étend horizontalement entre les appareils dentaires (28), et que l'élément détecteur (18) ou le marqueur de position (20) est fixé de manière dissociable au moyen d'attaches en fil (32) au fil de traitement (34).

8. Dispositif de mesure selon une des revendications 6 à 7, **caractérisé en ce**
**que** l'élément détecteur (18) ou le marqueur de position (20) est connecté au moyen d'un élément porteur (22) de manière dissociable aux appareils dentaires (28) ou au fil de traitement (34).
